# EUROPEAN PATENT APPLICATION

(11) **EP 4 659 679 A1**
(43) Date of publication of application: **10.12.2025**
(21) Application number: 24749766.2
(22) Date of filing: 03.02.2024
(51) Int. Cl.: A61B 6/03

(54) **STATIC REAL-TIME CT IMAGING SYSTEM AND IMAGING METHOD**

(30) Priority: 03.02.2023 CN 202310118999
(71) Applicant: Nanovision Technology (Beijing) Co., Ltd., Beijing 100094 (CN)
(72) Inventor: LI, Yunxiang, Beijing 100094 (CN); CAO, Hongguang, Beijing 100094 (CN); CUI, Zhili, Beijing 100094 (CN)
(74) Representative: Wang, Bo
(86) International application number: PCT/CN2024/075652
(87) International publication number: WO 2024/160291

(57) **Abstract**

Disclosed in the present invention are a static real-time CT imaging system and imaging method. The system comprises two ray source rings and a detector ring; on a Z axis, the left ray source ring and the right ray source ring are arranged on the left side and the right side of the detector ring; the left ray source ring and the right ray source ring are respectively annular multi-focus X-ray sources having a plurality of X-ray sources and are uniformly provided with a same number of focuses; the detector ring consists of a plurality of X-ray detectors arranged in a ring, and on the Z axis, the plurality of X-ray detectors jointly form a left detector sub-ring, a middle detector sub-ring and a right detector sub-ring; an X-ray source of the left ray source ring is placed between any two adjacent X-ray detectors of the left detector sub-ring; an X-ray source of the right ray source ring is placed between any two adjacent X-ray detectors of the right detector sub-ring; any two adjacent X-ray detectors of the middle detector sub-ring are in close contact. The use of the present invention can achieve a wider Z-direction coverage range, and effectively suppress cone-angle artifacts.

## Description

### BACKGROUND

### Technical Field

The present disclosure relates to a static real-time computed tomography (CT) imaging system and further relates to a corresponding static real-time CT imaging method, and belongs to the technical field of medical images.

### Related Art

CT is computed tomography. Its imaging principle is as follows: An X-ray beam and an X-ray detector with extremely high sensitivity are used to perform layer-by-layer cross-sectional scanning around a part of a human body. The X-ray detector receives X-rays transmitted through a current layer, converts the X-rays into an electrical signal by a photoelectric converter, amplifies the signal, then converts the signal into a digital signal by analog/digital conversion, and inputs the digital signal to a computer for processing. In the computer, the selected layer is divided into a plurality of cubes having the same volumes. The cubes are referred to as voxels. An X-ray attenuation coefficient or an X-ray absorption coefficient of each voxel is obtained by calculating information obtained by the layer-by-layer cross-sectional scanning, and then the X-ray attenuation coefficients or the X-ray absorption coefficients are arranged into a matrix, namely, a voxel digital matrix. Digital information in the voxel digital matrix is converted into small blocks with different gray scales from black to white, and the small blocks are referred to as pixels in two-dimensional projection and are arranged in a tomographic manner to form a CT image.

To increase a scanning speed, improve imaging precision and speed, avoid an impact on a centrifugal force caused by mechanical rotation, and reduce a signal tailing effect and an overlapping crosstalk degree during high-speed rotation, the applicant discloses a static real-time CT imaging system in Chinese Patent No. ZL201410425061.2. The static real-time CT imaging system includes an annular X-ray detector, an annular scanning X-ray source, and a scan timing sequence controller. In a scanning process of the static real-time CT imaging system, the annular scanning X-ray source does not need to rotate to a large extent, and X-ray projection positions are sequentially switched through electronic control, so that the scanning speed is increased by tens of times, and a dynamic three-dimensional image can be obtained: By using the X-ray detector, absorption data and energy data can be obtained, thereby implementing real-time data reconstruction.

In addition, a static real-time CT imaging system having paired ray source rings and an imaging control method are disclosed in Chinese Patent Application No. 201910865387.X. One ray source ring is arranged on each of a left side and a right side of a detector ring, and projection regions of the two ray source rings can be respectively reconstructed to obtain two reconstructed field of views (FOVs). However, there is a blank region between the two FOVs. Although the blank region can be supplemented in a spiral scan mode, the blank region cannot be supplemented in a single axial scan mode. Therefore, there is a limitation on an application of expanding scanning coverage.

### SUMMARY

A first technical problem to be solved by the present disclosure is to provide a static real-time CT imaging system.

Another technical problem to be solved by the present disclosure is to provide a static real-time CT imaging method.

To achieve the technical objectives, the present disclosure adopts the following technical solutions:
According to a first aspect of the embodiments of the present disclosure, a static real-time computed tomography CT imaging system is provided, at least including two ray source rings and one detector ring.

On a Z-axis, a left ray source ring and a right ray source ring are arranged on left and right sides of the detector ring, and the two ray source rings share one detector ring.

Each of the left ray source ring and the right ray source ring is an annular multi-focal X-ray source having a plurality of X-ray sources; the same quantity of focuses are uniformly arranged on the left ray source ring and the right ray source ring. The detector ring is composed of a plurality of X-ray detectors arranged into a ring, and on the Z-axis, the plurality of X-ray detectors jointly form a left detector sub-ring, a middle detector sub-ring, and a right detector sub-ring.

A first gap is formed between any two adjacent X-ray detectors on the left detector sub-ring, so as to place the X-ray sources of the left ray source ring. A second gap is formed between any two adjacent X-ray detectors on the right detector sub-ring, so as to place the X-ray sources of the right ray source ring. Any two adjacent X-ray detectors on the middle detector sub-ring are in close contact with each other.

Preferably, sizes of the plurality of X-ray detectors are the same. Meanwhile, the plurality of X-ray detectors are arranged in a staggered manner to form the left detector sub-ring, the middle detector sub-ring, and the right detector sub-ring. The focuses of the left ray source ring and the focuses of the right ray source ring are arranged in a relatively staggered manner.

Preferably, the X-ray detectors include a plurality of short-size X-ray detectors and a plurality of long-size X-ray detectors. The plurality of short-size X-ray detectors and the plurality of long-size X-ray detectors are sequentially crosswise arranged to form the left detector sub-ring, the middle detector sub-ring, and the right detector sub-ring. The left ray source ring and the right ray source ring are symmetrically arranged on the left and right sides of the detector ring.

Preferably, the X-ray detectors include a main detector and a scatter detector. The scatter detector is arranged on one side or two sides of the main detector.

A beam collimator is arranged between the X-ray sources and the X-ray detectors, to limit ray beam coverage ranges of the X-ray sources to cover the main detector only.

Preferably, the beam collimator specifically includes:
a Z-direction beam collimation unit, which is capable of doing an opening or closing motion on the Z-axis, so as to limit coverage ranges of ray beams of the X-ray sources in a Z-direction; and
an X-direction beam collimation unit, which is capable of doing an opening or closing motion on an X-axis, so as to limit coverage ranges of ray beams of the X-ray sources in an X-direction.

The Z-direction is an axial direction of the detector ring, and the X-direction is a tangent direction of the detector ring.

Preferably, the Z-direction beam collimation unit includes a Z-direction fixed portion and a Z-direction movable portion; the Z-direction fixed portion is fixedly arranged along the Z-axis. The Z-direction movable portion approaches or moves away from the Z-direction fixed portion along the Z-axis, to adjust a size of an opening between the Z-direction fixed portion and the Z-direction movable portion.

The X-direction beam collimation unit includes two X-direction movable portions. The two X-direction movable portions approach or move away from each other along the X-axis, so as to adjust a size of an opening between the two X-direction movable portions.

Preferably, the plurality of X-ray sources in both the left ray source ring and the right ray source ring perform axial scan in any one of the following manners:
(1) one ray source on the left side is exposed, one ray source on the right side is exposed, then one ray source on the left side is exposed, ..., and so on; in this way, all the ray sources are exposed by turns in a left-and-right alternation manner, and projected images of all the ray sources are obtained;
(2) K ray sources on the left side are simultaneously exposed, and X-ray detectors corresponding to light field coverage ranges of the K ray sources on the left side are not shared; then, K ray sources on the right side are simultaneously exposed, and X-ray detectors corresponding to light field coverage ranges of the K ray sources on the right side are not shared;...; and so on; in this way, all the ray sources are exposed by turns in a left-and-right alternation manner, and projected images of all the ray sources are obtained, where K is a positive integer and is not less than 2;
(3) the ray sources on the left side are exposed by turns, and only projected images of the ray sources on the left side are obtained; and
(4) the ray sources on the right side are exposed by turns, and only projected images of the ray sources on the right side are obtained.

Preferably, the static real-time CT imaging system further includes a scan timing sequence controller. Exposure timing sequences of the plurality of focuses in both the left ray source ring and the right ray source ring and acquisition timing sequences of the corresponding X-ray detectors in the detector ring are controlled by the scan timing sequence controller.

According to a second aspect of the embodiments of the present disclosure, a static real-time CT imaging method is provided, which is implemented based on the static real-time CT imaging system and at least includes the following steps:
controlling, by the scan timing sequence controller, the X-ray sources in both the left ray source ring and the right ray source ring and the X-ray detectors, corresponding to the left ray source ring and the right ray source ring, in the detector ring to operate in a predetermined scan timing sequence;
emitting, by the plurality of X-ray sources in both the left ray source ring and the right ray source ring, X-rays according to a predetermined exposure timing sequence; and acquiring, by the corresponding X-ray detectors, projection information of the X-rays on the X-ray detectors after the X-rays are transmitted through a tested object.

Preferably, the projection information includes left-side partial projection information acquired by the left detector sub-ring, complete projection information acquired by the middle detector sub-ring, and right-side partial projection information acquired by the right detector sub-ring.

Compared with the prior art, the present disclosure has the following technical characteristics:
1. Because of the structure with the two ray source rings, a scan coverage range in the Z-axis direction is enlarged, so that there is no blank region within the left and right Z-direction coverage ranges. Single axial scan can implement a wider Z-direction coverage range, and can further reduce a projection cone angle, thereby effectively suppressing a cone-beam artifact.
2. The plurality of X-ray sources (namely, the plurality of focuses) in both the left ray source ring and the right ray source ring can implement axial scan in various manners, to meet different scanning requirements. In addition, the plurality of focuses in both the left ray source ring and the right ray source ring can implement spectral scan in various manners.
3. The X-ray detector includes a scatter detector and a main detector, so that scattering correction can be implemented by measuring a scattering signal distribution by using the scatter detector.
4. The beam collimator can adjust the ray beam coverage ranges of the X-ray sources, to meet different FOV range requirements.

### BRIEF DESCRIPTION OF THE DRAWINGS

FIG. 1 is a schematic diagram of a three-dimensional structure of a static real-time CT imaging system according to a first embodiment of the present disclosure;
FIG. 2 is a partial top view of a static real-time CT imaging system according to a first embodiment of the present disclosure;
FIG. 3 is a schematic diagram of a projection of a structure with a single ray source ring in the existing art;
FIG. 4 is a schematic diagram of a projection of a structure with two ray source rings according to a first embodiment of the present disclosure;
FIG. 5 is a diagram of a projection relationship between an X-ray beam and a detector ring in a state that detector sub-rings do not include a detector sub-ring Z_{L} in a Z-axis direction according to a first embodiment of the present disclosure;
FIG. 6 is a diagram of a projection relationship between an X-ray beam and a detector ring that detector sub-rings do not include a detector sub-ring Z_{R} in a Z-axis direction according to a first embodiment of the present disclosure;
FIG. 7 is a diagram of a projection relationship between an X-ray beam and a detector ring in a state that no detector sub-ring is missing in a Z-axis direction according to a first embodiment of the present disclosure;
FIG. 8 is a diagram of a projection relationship between an X-ray beam and a detector ring in a state that no detector sub-ring is missing in an X-axis direction according to a first embodiment of the present disclosure;
FIG. 9 is a diagram of a projection relationship between an X-ray beam and a detector ring in a state of missing Z_{L} or Z_{R} of a detector sub-ring in an X-axis direction according to a first embodiment of the present disclosure;
FIG. 10 is a schematic diagram of a timing sequence of sequentially emitting X-rays by single X-ray sources according to a first embodiment of the present disclosure;
FIG. 11 is a schematic diagram of a timing sequence of synchronously emitting X-rays by three X-ray sources according to a first embodiment of the present disclosure;
FIG. 12 is a schematic diagram of a timing sequence of synchronously emitting X-rays by six X-ray sources according to a first embodiment of the present disclosure;
FIG. 13 is a schematic structural diagram of an X-ray detector according to a first embodiment of the present disclosure;
FIG. 14 is a schematic diagram of a beam collimator between an X-ray source and an X-ray detector according to a first embodiment of the present disclosure;
FIG. 15 is a schematic structural diagram of arrangement of scatter detectors in a Z-X plane by using a sparse matrix according to a first embodiment of the present disclosure;
FIG. 16 is a diagram of a beam collimation principle of a Z-direction beam collimation unit according to a first embodiment of the present disclosure;
FIG. 17 is a diagram of a beam collimation principle of a Z-direction beam collimation unit according to a first embodiment of the present disclosure; and
FIG. 18 is a partial top view of a static real-time CT imaging system according to a second embodiment of the present disclosure.

### DETAILED DESCRIPTION

Technical contents of the present disclosure will be described in detail below with reference to the accompanying drawings and specific embodiments.

### First embodiment

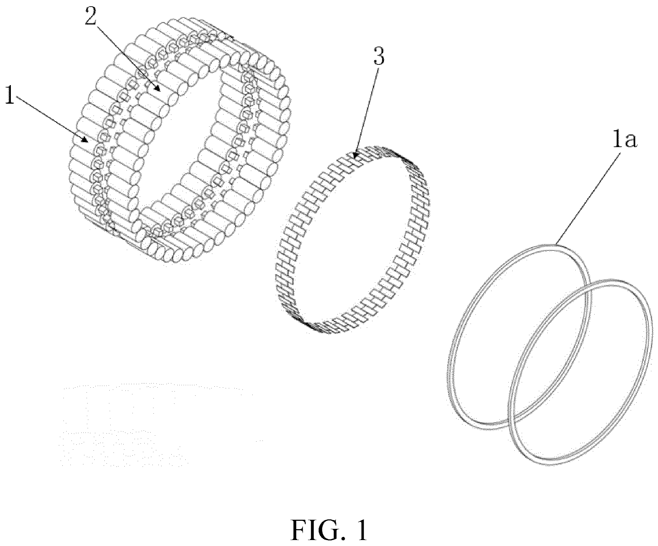

As shown in FIG. 1, a static real-time CT imaging system provided by the first embodiment of the present disclosure at least includes two ray source rings, a detector ring 3, and a scan timing sequence controller. In a Z-axis direction, a left ray source ring 1 and a right ray source ring 2 are arranged on left and right sides of the detector ring 3, and the two ray source rings share one detector ring 3. The left ray source ring 1, the right ray source ring 2, and the detector ring 3 are mounted on a rotary support through a bearing 1a, and are located on the same axis which is a Z-axis commonly referred to in the CT field.

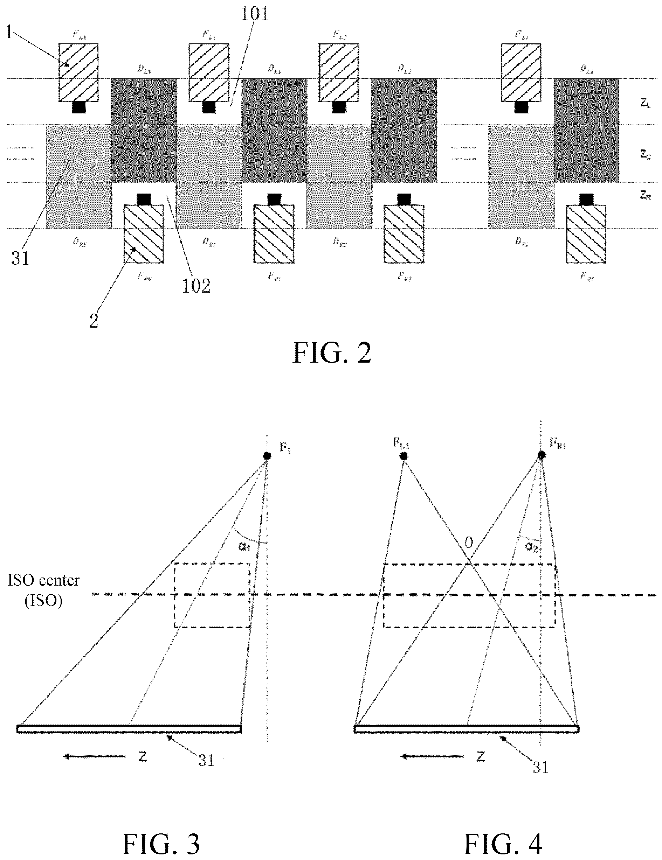

Specifically, in this embodiment, each of the left ray source ring 1 and the right ray source ring 2 is an annular multi-focal X-ray source having a plurality of X-ray sources. N focuses are uniformly arranged on each of the left ray source ring 1 and the right ray source ring 2. The two ray source rings have 2N focuses in total. The focuses of the left ray source ring 1 are denoted as F_{Li}, and the focuses of the right ray source ring 2 are denoted as F_{Ri}, where i belongs [1, N], and the N focuses F_{Li} and the N focuses F_{Ri} are arranged in a relatively staggered manner. As shown in FIG. 2, the detector ring 3 is composed of a plurality of X ray detectors 31 arranged into a ring. Furthermore, sizes of the plurality of X-ray detectors are the same. The plurality of X-ray detectors 31 are arranged in a staggered manner to form a left detector sub-ring Z_{L}, a middle detector sub-ring Z_{C}, and a right detector sub-ring Z_{R}. Specifically, a first gap 101 is formed between any two adjacent X-ray detectors on the left detector sub-ring Z_{L}, so as to place an X-ray source of the left ray source ring 1. A second gap 102 is formed between any two adjacent X-ray detectors on the right detector sub-ring Z_{R}, so as to place an X-ray source of the right ray source ring 2. Any two adjacent X-ray detectors on the middle detector sub-ring Z_{C} are in close contact with each other.

Exposure timing sequences of the plurality of focuses in both the left ray source ring 1 and the right ray source ring 2 and data acquisition timing sequences of the corresponding X-ray detectors in the detector ring 3 are controlled by the same scan timing sequence controller. The plurality of X-ray sources in both the left ray source ring 1 and the right ray source ring 2 emit X-rays according to a predetermined exposure timing sequence, and the X-ray detectors 31, corresponding to the X-ray sources, in the detector ring 3 acquire projection information of the X-rays on the X-ray detectors 31 after the X-rays are transmitted through a measured object. It can be understood that, the projection information in this embodiment includes left-side partial projection information acquired by the left detector sub-ring Z_{L}, complete projection information acquired by the middle detector sub-ring Z_{C}, and right-side partial projection information acquired by the right detector sub-ring Z_{R}. Thus, because of the structure with the two ray source rings, a scan coverage range in the Z-axis direction is enlarged, so that there is no blank region within the left and right Z-direction coverage ranges. Single axial scan can implement a wider Z-direction coverage range, and can further reduce a projection cone angle, thereby effectively suppressing a cone-beam artifact.

A difference between a single-ray-source structure and a double-ray-source structure is described in detail below:
FIG. 3 is a schematic diagram of a Z-direction projection of a structure with a single ray source ring in the existing art. FIG. 4 is a schematic diagram of a Z-direction projection of a structure with two ray source rings according to this embodiment. Where Fᵢ is a focus of a single ray source ring, and F_{Li} and F_{Ri} are two focuses of the structure with the two ray source rings in this embodiment. The rectangular dashed line region is an image range that can be reconstructed in the Z-direction corresponding to the two types of structures; and α₁ and α₂ respectively correspond to cone angles of projections in two modes. It can be known with reference to FIG. 3 and FIG. 4 that in a case that a Z-direction width in of a detector keeps unchanged and a vertical distance between a focus and the detector keeps unchanged, α₁>α₂ and a Z-direction coverage range in FIG. 3 < a Z-direction coverage range in FIG. 4. That is: In this embodiment, the Z-direction coverage range is larger and a cone angle of a ray beam is smaller, so that a problem of a cone-beam artifact during image reconstruction can be reduced.

In addition, it can be understood that in this embodiment, a scan coverage range in the Z-axis direction is enlarged by using the structure with the two ray source rings. It can be learned with reference to FIG. 4 that there is a larger range for an image that can be reconstructed in the Z-direction, to ensure a requirement of a clinic application for Z-direction coverage of a CT machine.

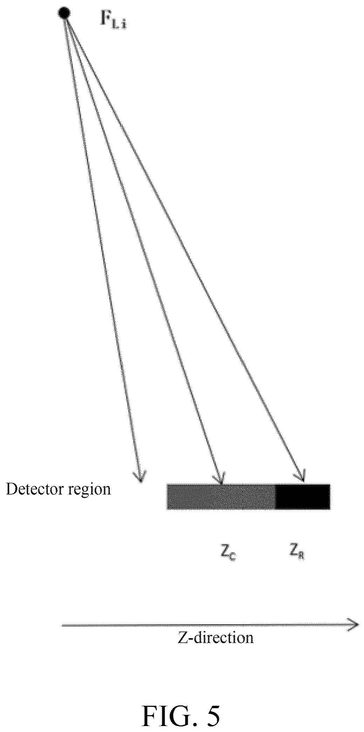

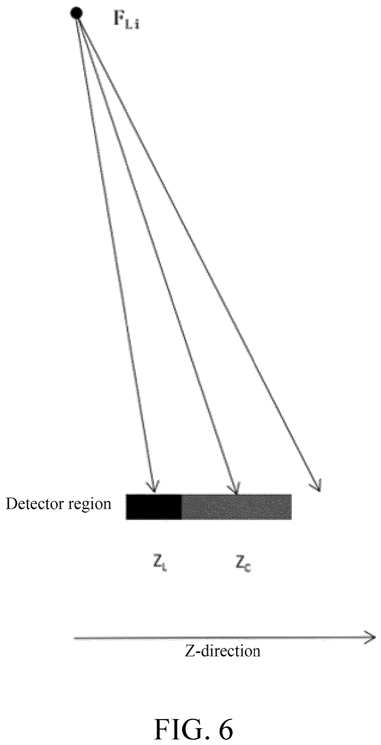

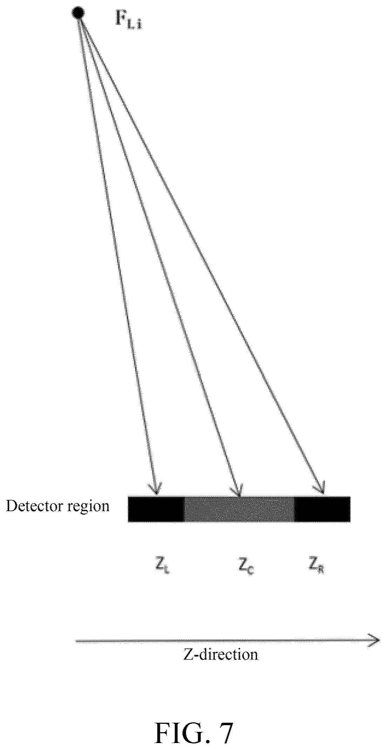

Referring to FIG. 5 to FIG. 7, in the Z-axis direction, an X-ray beam and ZL, ZC, and ZR of a detector ring form three correspondences. Specifically, when a photosensitive element of a ZL region of the detector ring is missing, the X-ray beam and ZC and ZR regions of the X-ray detector form a projection relationship shown in FIG. 5. Similarly, when a photosensitive element of the ZR region of the detector ring is missing, the X-ray beam and ZC and ZL regions of the X-ray detector form a projection relationship shown in FIG. 6. When the X-ray detector completely has the photosensitive elements of the ZL, ZC, and ZR regions, the ray beam and the detector ring form a projection relationship shown in FIG. 7.

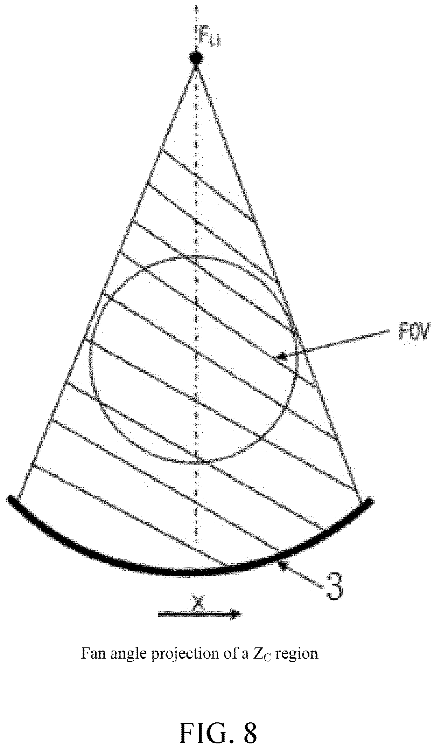

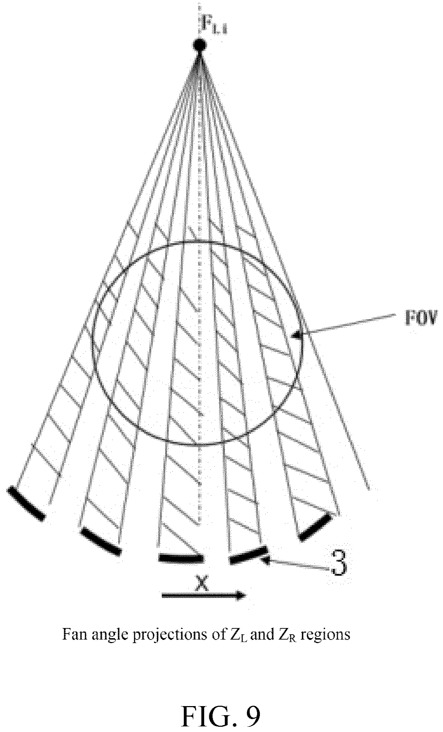

Referring to FIG. 8 and FIG. 9, in an X-axis direction, an X-ray beam and Z_{L}, Z_{C}, and Z_{R} of a detector ring form two possible correspondences, where the X-axis direction is a tangent direction of the detector ring 3. Specifically, as shown in FIG. 8, in an X-ray projection, the oblique line segments from the top left side to the bottom right side show an actual X-ray projection geometry. A projection geometric relationship corresponding to the Z_{C} region of the detector ring is a projection for full FOV coverage. As shown in FIG. 9, when the Z_{L} region and the Z_{R} region of the detector ring are projected by the ray sources, since the photosensitive elements of the detectors are partially missing, projections of the Z_{L} and Z_{R} regions of the detector ring are missing in the X-axis direction (namely, the tangent direction). In this embodiment, partial missing of this projection may be solved by using a sparse sampling reconstruction algorithm, thereby implementing completeness of an FOV of a reconstructed image in the X-direction.

In the above embodiments, the X-ray sources (namely, the plurality of focuses) in both the left ray source ring 1 and the right ray source ring 2 may implement axial scan in the following various manners. Specifically, at least the following four axial scan manners are included:
(1) One ray source on the left side is exposed; then one ray source on the right side is exposed; then one ray source on the left side is exposed;...; and so on. In this way, all the ray sources are exposed by turns in a left-and-right alternation manner, and projected images of all the ray sources are obtained.
(2) A group of K ray sources on the left side are simultaneously exposed, and X-ray detectors 31 corresponding to light field coverage ranges of the K ray sources on the left side are not shared. Then, a group of K ray sources on the right side are simultaneously exposed, and X-ray detectors 31 corresponding to light field coverage ranges of the K ray sources on the right side are not shared. Then, another group of ray sources on the left side are simultaneously exposed;...; and so on. In this way, all the ray sources are exposed by turns in a left-and-right alternation manner, and projected images of all the ray sources are obtained. where K is a positive integer and is not less than 2.
(3) The ray sources on the left side are exposed by turns, and only projected images of the ray sources on the left side are obtained.
(4) The ray sources on the right side are exposed by turns, and only projected images of the ray sources on the right side are obtained.

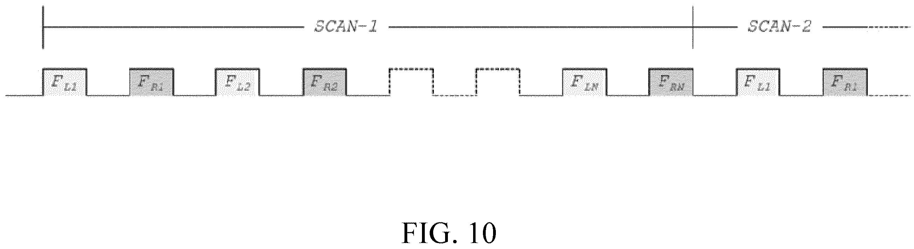

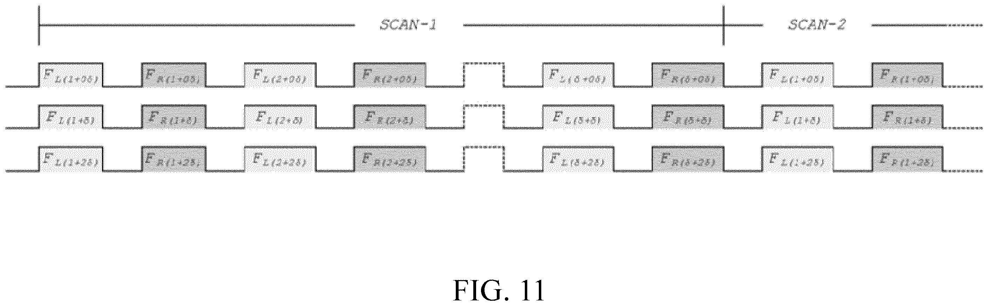

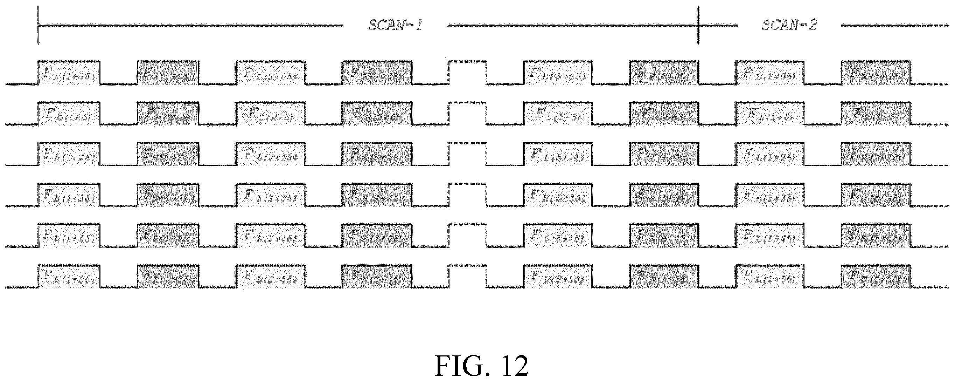

In addition, in the axial scan manners (1), (2), (3), and (4), an exposure timing sequence of one or more focuses in different regions of the single ray source ring may further use a scan timing sequence of simultaneous or time-division operation, point-by-point scanning or dot interlaced scanning, or row-by-row or interlaced transmission of X-rays. The X-ray sources in both the left ray source ring 1 and the right ray source ring 2 may sequentially emit X-rays in a circumferential direction, or may sequentially emit X-rays at an interval of a plurality of X-ray sources. Only one of the X-ray sources in both the left ray source ring 1 and the right ray source ring 2 may emit X-rays (referring to FIG. 10), or a plurality of X-ray sources may simultaneously emit X-rays (referring to FIG. 11 and FIG. 12). A maximum quantity of X-ray sources that concurrently emit X-rays is based on satisfying that the X-rays that are concurrently emitted do not interfere with each other on the X-ray detectors 31. The X-ray sources that concurrently emit the X-rays are preferably uniformly distributed on a circumference.

The X-rays emitted by the X-ray sources in both the left ray source ring 1 and the right ray source ring 2 irradiate the corresponding X-ray detectors 31 in the detector ring 3 after being transmitted through a tested object. A data acquisition and processing unit is composed of a plurality of distributed subsystems, and an embedded graphic processing unit (GPU) is integrated into each subsystem. X-ray projection information received by the X-ray detectors 31 is acquired by the data acquisition and processing unit for image reconstruction. Reconstructed image information is then transmitted to an image data storage unit and a human-computer interaction unit, to complete storage and visual reproduction of an image in the image data storage unit and the human-computer interaction unit. Certainly, an existing CT data acquisition and processing manner may alternatively be used: The data acquisition and processing unit only acquires data, and then transmits the data to an image reconstruction unit and a data storage unit for reconstruction and storage.

The static real-time CT imaging system may implement various spectral scan manners by using the plurality of focuses in both the left ray source ring 1 and the right ray source ring 2. First, the left ray source ring 1 and the right ray source ring 2 may perform spectral scan by instant energy level switching of single X-ray sources, and may instantly switch various energy levels (such as switching between 80 kV, 100 kV, 120 kV, and 140 kV). A specific quantity of energy levels for being switched is determined by a design requirement. After a particular X-ray source performs spectral scan by instant energy level switching, a next X-ray source under the control of the scan timing sequence performs spectral scan in the same manner, until the entire scan work is completed. Secondly, the left ray source ring 1 and the right ray source ring 2 may perform spectral scan by circular intermittent energy level switching. That is, after the X-ray sources of both the left ray source ring 1 and the right ray source ring 2 complete one instance of circular scan at the same energy level under the control of the timing sequence, the X-ray sources all switch to another energy level and repeatedly complete a next instance of circular scan, until switching of all the energy levels is completed. Thirdly, the left ray source ring 1 and the right ray source ring 2 may alternatively perform circular multi-spectral scan. That is, X-ray sources for scanning that are distributed on a circumference are divided into a plurality of groups, and each group is unified at an energy level. After one instance of circular scan is completed under the control of the timing sequence, the energy levels of the groups of X-ray sources for scanning are then respectively switched to corresponding next energy levels, to repeatedly complete a next instance of circular scan, until switching of all the energy levels is completed. Finally, the left ray source ring 1 and the right ray source ring 2 may alternatively use two different energy levels at the same time. Specifically, the plurality of focuses in the left ray source ring 1 use one energy level (A kV), and the plurality of focuses in the right ray source ring 2 use the other energy level (B kV, B≠A). Spectral images of the two energy levels are implemented in one instance of scan.

It should be noted that, each X-ray detector 31 in this embodiment may be a photon stream detector or may be replaced with an energy integrating detector, to implement the same function.

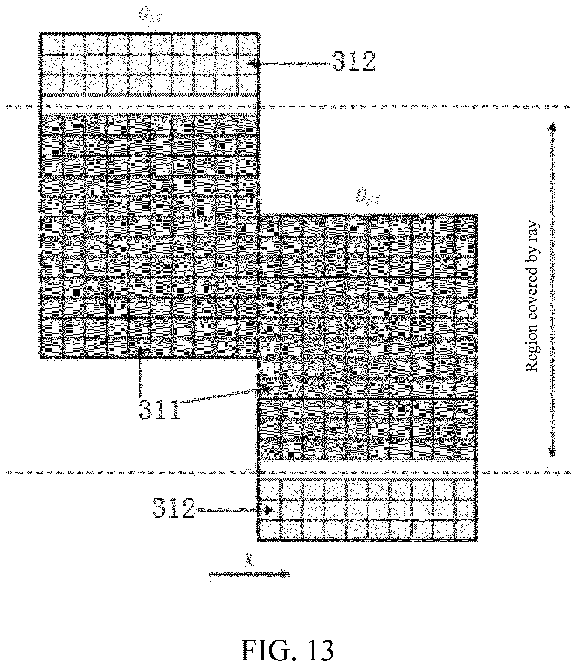

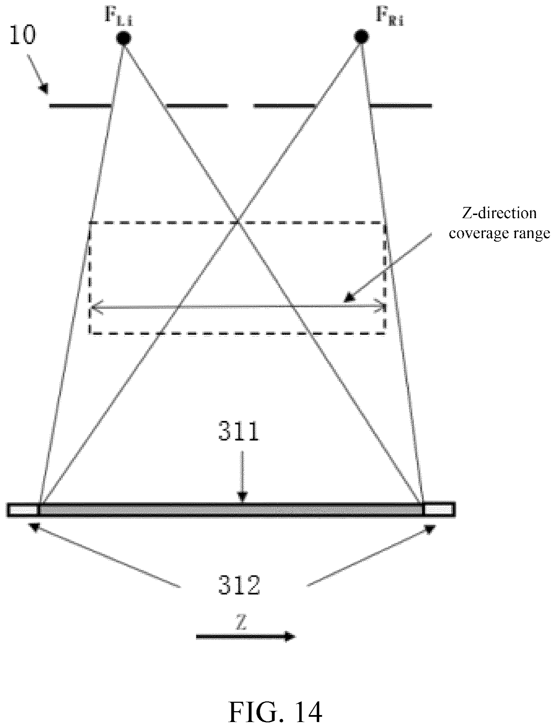

Referring to FIG. 13, in the above embodiments, preferably, the X-ray detectors 31 include main detectors 311 and scatter detectors 312. Each scatter detector 312 is arranged on one side or two sides of each main detector 311. Moreover, as shown in FIG. 14, in this embodiment, a beam collimator 10 is arranged between the X-ray sources and the X-ray detectors 31, to limit main ray beam coverage ranges of the X-ray sources, so that the main ray beam coverage ranges of the X-ray sources only cover the main detectors 311. Therefore, the scatter detectors 312 are not covered by a main ray beam, and signals measured by the scatter detectors 312 are X-ray scattering distributions generated after the main ray beam interacts with a scanned object. Therefore, an amount of scattering distribution information is obtained while a projected image is obtained, thus forming a scattering matrix. The scattering matrix is integrated with a projection matrix obtained by the main detector, and is uploaded to a data acquisition server in a data stream manner. Thus, the data acquisition server separates scattering information from image information, restores complete scattering information, and removes the scattering information from the image information, thereby implementing scattering correction.

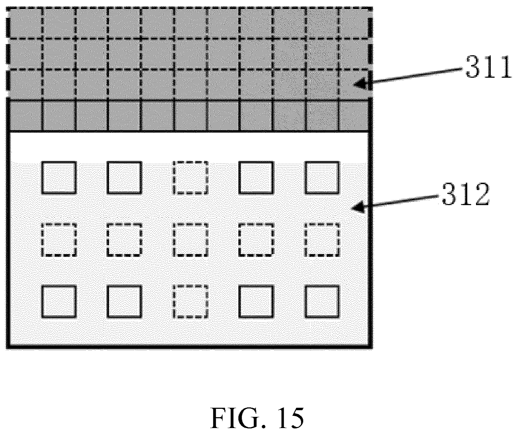

It can be understood that in the above embodiments, the scatter detectors 312 and the main detectors 311 do not depend on a specific implementation form, and may have various implementation forms. For example: They may be independent photodiode (PD) arrays, or may be various detector photosensitive element arrays (including but not limited to CMOS + PD, TFT + PD, indirect X-ray linear detectors, and direct X-ray detectors, such as: amorphous selenium, cadmium telluride (CdTe), tellurium zinc cadmium (CdZnTe, CZT), cadmium selenide (CdSe), and IGZO). In this embodiment, pixels of the main detectors 311 are uniformly distributed. A pixel matrix of each main detector 311 may be represented as I(R*M), namely, R rows and M columns. A pixel matrix of each scatter detector 312 may be represented as S(r*m), namely, r rows and m columns. When m=M, a quantity of columns of the main detector is consistent with a quantity of columns of the scatter detector. When m<M, the scatter detector 312 may be arranged on a Z-X plane in a sparse arrangement form (shown in FIG. 15). Because of the sparse arrangement of the scatter detector 312, with reference to a low-frequency characteristic of a scattering signal, scatterometry-based scattering estimation is performed in the X-direction and the Z-direction, and the scattering signal is removed from the image information. The method includes step S10 to step S30:
S10: Measure and supplement scattering signals in the X-direction.

In a single X-ray detector 31, in the X-direction, the scattering signals may be supplemented by interpolation, to obtain scattering matrix columns in the same quantity as the quantity of the columns of the main detector, and the scattering signals of S(r*m) are corrected to S(r*M).

Between the X-ray detectors 31, in the X-direction, since D_{Li} and D_{Ri} are not aligned in the Z-direction, a scattering amount of D_{Li} may be supplemented by interpolation by using a scattering amount of D_{Ri}. Similarly, the scattering amount of D_{Ri} may be supplemented by interpolation by using the scattering amount of D_{Li}.

S20: Measure and supplement scattering signals in the Z-direction.

In the Z-direction, the quantity r of rows of scatter detectors arranged on two sides needs to be greater than or equal to 3, and the scatter detectors need to avoid a penumbra region of the main ray. Scattering signals on image columns in the entire Z-direction are fitted according to distribution trends of the scattering signals on the two sides, thus obtaining a scattering signal matrix S(R*M).

S30: Remove the scattering signals.

The image matrix I (R*M) is subtracted by the extracted scattering signal matrix S(R*M), to remove scattering information.

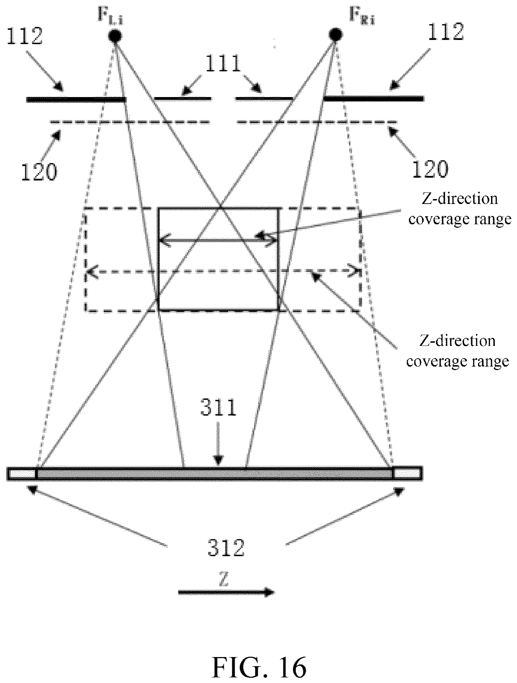

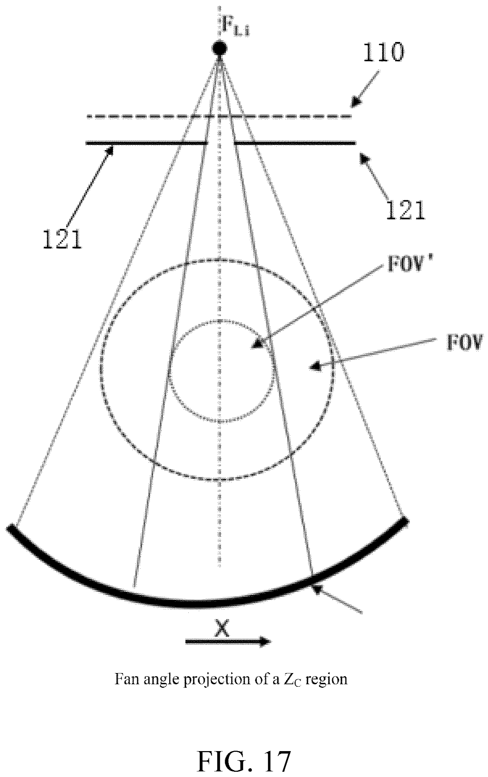

As shown in FIG. 16 and FIG. 17, in the above embodiments, the beam collimator 10 includes: a Z-direction beam collimation unit 110 and an X-direction beam collimation unit 120. The Z-direction beam collimation unit 110 is capable of doing an opening or closing motion in the Z-direction, to limit ray beam coverage ranges of the X-ray sources in the Z-direction. The X-direction beam collimation unit 120 is capable of doing an opening or closing motion in the X-direction, so as to limit ray beam coverage ranges of the X-ray sources in the X-direction. Specifically, in this embodiment, as shown in FIG. 16, the Z-direction beam collimation unit 110 includes a Z-direction fixed portion 111 and a Z-direction movable portion 112. The Z-direction fixed portion 111 is fixedly arranged along the Z-axis, and the Z-direction movable portion 112 approaches or moves away from the Z-direction fixed portion 111 along the Z-axis, to adjust a size of an opening between the Z-direction fixed portion 111 and the Z-direction movable portion 112. Similarly, as shown in FIG. 17, the X-direction beam collimation unit 120 includes two X-direction movable portions 121. The two X-direction movable portions 121 approach or move away from each other along the X-axis, to adjust a size of an opening between the two X-direction movable portions 121. It can be understood that a larger opening indicates larger ray beam coverage ranges (i.e., FOV ranges) of the X-ray sources; and a smaller opening indicates smaller ray beam coverage ranges (i.e., FOV ranges) of the X-ray sources, so that adaptive adjustment may be performed according to actual clinic requirements.

In conclusion, the static real-time CT imaging system provided by the first embodiment of the present disclosure has the following beneficial effects:
1. A scan coverage range in the Z-axis direction is enlarged, so that there is no blank region within the left and right Z-direction coverage ranges. Single axial scan can implement a wider Z-direction coverage range, and can further reduce a projection cone angle, thereby effectively suppressing a cone-beam artifact.
2. The X-ray sources (namely, the plurality of focuses) in both the left ray source ring 1 and the right ray source ring 2 can implement axial scan in various manners, to meet different scanning requirements. In addition, the plurality of focuses in both the left ray source ring 1 and the right ray source ring 2 implement spectral scan in various manners.
3. The X-ray detectors 31 include the scatter detectors 312 and the main detectors 311, so that scattering correction can be implemented by using the scatter detectors 312.
4. The beam collimator 10 can adjust the ray beam coverage ranges of the X-ray sources, to meet different FOV range requirements.

### Second embodiment

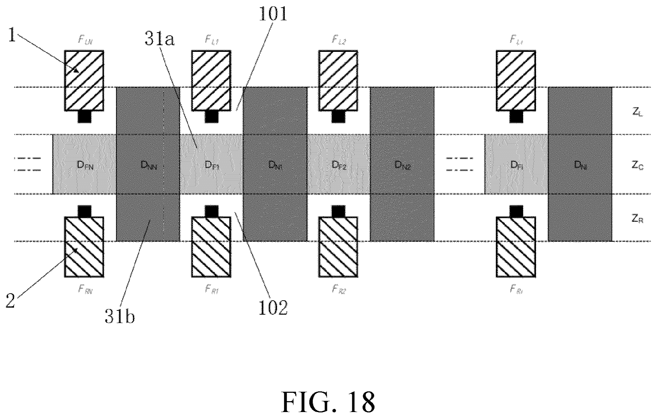

As shown in FIG. 18, a static real-time CT imaging system provided by the second embodiment of the present disclosure includes two ray source rings, a detector ring 3, and a scan timing sequence controller. Compared with the first embodiment, this embodiment has a different arrangement manner for the ray source rings and the detector ring 3.

Specifically, in this embodiment, the plurality of X-ray detectors include a plurality of short-size X-ray detectors 31a and a plurality of long-size X-ray detectors 31b. The plurality of short-size X-ray detectors 31a and the plurality of long-size X-ray detectors 31b are sequentially crosswise arranged to form a left detector sub-ring, a middle detector sub-ring, and a right detector sub-ring. Furthermore, the left ray source ring 1 and the right ray source ring 2 are symmetrically arranged on left and right sides of the detector ring 3.

It can be understood that in this embodiment, during X-ray exposure scanning, the short-size X-ray detectors 31a may only measure a projection of a region in which the middle detector sub-ring is located, and the long-size X-ray detectors 31b may measure projections of all regions in which the left detector sub-ring, the middle detector sub-ring, and the right detector sub-ring are located. That is: Z-direction expansion is implemented in a region in which the long-size X-ray detectors 31b are located, which is beneficial to improving imaging quality.

In addition, compared with the first embodiment, due to space occupation by the X-ray sources in this embodiment, when the quantity N of the focuses of the X-ray sources increases, a corresponding radian interval between the focuses decreases, so that the region in which the long-size X-ray detectors 31b are located gradually decreases. If the region in which the long-size X-ray detectors 31b are located disappears, the Z-direction expansion function is lost. Therefore, the quantity N of the focuses of the X-ray sources needs to be adaptively set according to requirements. In addition, the layout manner in the first embodiment can achieve a complementary effect on the projected images. There is no complementary effect on the projected images in this embodiment. The imaging effect of this embodiment is slightly worse than that of the first embodiment, but this embodiment can still implement the Z-direction expansion. Compared with a CT imaging system without Z-direction expansion in the existing art, this embodiment has a better imaging effect.

Other structures except the above structure in this embodiment are the same as those in the first embodiment, and will not be elaborated here.

### Third embodiment

A third embodiment of the present disclosure provides a static real-time CT imaging method, which is implemented based on the static real-time CT imaging system in the first embodiment or the second embodiment and at least includes step S1 to step S2:
S1: The scan timing sequence controller controls the X-ray sources in both the left ray source ring 1 and the right ray source ring 2 and the X-ray detectors 31, corresponding to the left ray source ring 1 and the right ray source ring 2, in the detector ring 3 to operate in a predetermined scan timing sequence.
S2: The plurality of X-ray sources in both the left ray source ring 1 and the right ray source ring 2 emit X-rays according to a predetermined exposure timing sequence, and the corresponding X-ray detectors 31 acquire projection information of the X-rays on the X-ray detectors 31 after the X-rays are transmitted through a tested object.

It can be understood that, the projection information in this embodiment includes left-side partial projection information acquired by the left detector sub-ring, complete projection information acquired by the middle detector sub-ring, and right-side partial projection information acquired by the right detector sub-ring.

The above describes in detail the static real-time CT imaging system and the static real-time CT imaging method that are provided by the present disclosure. For those of ordinary skill in the art, any obvious changes made to the present disclosure without deviating from its substantive content will constitute infringement of the patent rights of the present disclosure, and they will bear corresponding legal responsibilities.

## Claims

1. A static real-time computed tomography (CT) imaging system, at least comprising two ray source rings and one detector ring, wherein
on a Z-axis, a left ray source ring and a right ray source ring are arranged on left and right sides of the detector ring, and the two ray source rings share one detector ring;
each of the left ray source ring and the right ray source ring is an annular multi-focal X-ray source having a plurality of X-ray sources; the same quantity of focuses are uniformly arranged on the left ray source ring and the right ray source ring; the detector ring is composed of a plurality of X-ray detectors arranged into a ring, and on the Z-axis, the plurality of X-ray detectors jointly form a left detector sub-ring, a middle detector sub-ring, and a right detector sub-ring;
a first gap is formed between any two adjacent X-ray detectors on the left detector sub-ring, so as to place the X-ray sources of the left ray source ring; a second gap is formed between any two adjacent X-ray detectors on the right detector sub-ring, so as to place the X-ray sources of the right ray source ring; and any two adjacent X-ray detectors on the middle detector sub-ring are in close contact with each other.

2. The static real-time CT imaging system according to claim 1, wherein
sizes of the plurality of X-ray detectors are the same;
the plurality of X-ray detectors are arranged in a staggered manner to form the left detector sub-ring, the middle detector sub-ring, and the right detector sub-ring; and
the focuses of the left ray source ring and the focuses of the right ray source ring are arranged in a relatively staggered manner.

3. The static real-time CT imaging system according to claim 1, wherein
the X-ray detectors comprise a plurality of short-size X-ray detectors and a plurality of long-size X-ray detectors; the plurality of short-size X-ray detectors and the plurality of long-size X-ray detectors are sequentially crosswise arranged to form the left detector sub-ring, the middle detector sub-ring, and the right detector sub-ring; and the left ray source ring and the right ray source ring are symmetrically arranged on the left and right sides of the detector ring.

4. The static real-time CT imaging system according to claim 1, wherein
the X-ray detectors comprise a main detector and a scatter detector; the scatter detector is arranged on one side or two sides of the main detector; and
a beam collimator is arranged between the X-ray sources and the X-ray detectors, to limit ray beam coverage ranges of the X-ray sources to cover the main detector only.

5. The static real-time CT imaging system according to claim 4, wherein the beam collimator comprises:
a Z-direction beam collimation unit, which is capable of doing an opening or closing motion on the Z-axis, so as to limit ray beam coverage ranges of the X-ray sources in a Z-direction; and
an X-direction beam collimation unit, which is capable of doing an opening or closing motion on an X-axis, so as to limit ray beam coverage ranges of the X-ray sources in an X-direction; and
the Z-direction is an axial direction of the detector ring, and the X-direction is a tangent direction of the detector ring.

6. The static real-time CT imaging system according to claim 5, wherein
the Z-direction beam collimation unit comprises a Z-direction fixed portion and a Z-direction movable portion; the Z-direction fixed portion is fixedly arranged along the Z-axis; the Z-direction movable portion approaches or moves away from the Z-direction fixed portion along the Z-axis, to adjust a size of an opening between the Z-direction fixed portion and the Z-direction movable portion;
the X-direction beam collimation unit comprises two X-direction movable portions; and the two X-direction movable portions approach or move away from each other along the X-axis, so as to adjust a size of an opening between the two X-direction movable portions.

7. The static real-time CT imaging system according to claim 1, wherein the plurality of X-ray sources in both the left ray source ring and the right ray source ring perform axial scan in any one of the following manners:
(1) one ray source on the left side is exposed, then one ray source on the right side is exposed, and then one ray source on the left side is exposed, so that all the ray sources are exposed by turns in a left-and-right alternation manner, and projected images of all the ray sources are obtained;
(2) K ray sources on the left side are simultaneously exposed, and X-ray detectors corresponding to light field coverage ranges of the K ray sources on the left side are not shared; then, K ray sources on the right side are simultaneously exposed, and X-ray detectors corresponding to light field coverage ranges of the K ray sources on the right side are not shared, so that all the ray sources are exposed by turns in a left-and-right alternation manner, and projected images of all the ray sources are obtained; wherein K is a positive integer and is not less than 2;
(3) the ray sources on the left side are exposed by turns, and only projected images of the ray sources on the left side are obtained; and
(4) the ray sources on the right side are exposed by turns, and only projected images of the ray sources on the right side are obtained.

8. The static real-time CT imaging system according to claim 1, further comprising a scan timing sequence controller, wherein exposure timing sequences of the plurality of focuses in both the left ray source ring and the right ray source ring and acquisition timing sequences of the corresponding X-ray detectors in the detector ring are controlled by the scan timing sequence controller.

9. A static real-time CT imaging method, implemented based on the static real-time CT imaging system according to any one of claims 1 to 8, and comprising the following steps:
controlling, by the scan timing sequence controller, the X-ray sources in both the left ray source ring and the right ray source ring and the X-ray detectors, corresponding to the left ray source ring and the right ray source ring, in the detector ring to operate in a predetermined scan timing sequence;
emitting, by the plurality of X-ray sources in both the left ray source ring and the right ray source ring, X-rays according to a predetermined exposure timing sequence; and acquiring, by the corresponding X-ray detectors, projection information of the X-rays on the X-ray detectors after the X-rays are transmitted through a tested object.

10. The static real-time CT imaging method according to claim 9, wherein
the projection information comprises left-side partial projection information acquired by the left detector sub-ring, complete projection information acquired by the middle detector sub-ring, and right-side partial projection information acquired by the right detector sub-ring.
